# EUROPEAN PATENT SPECIFICATION

(11) **EP 2 451 396 B1**
(45) Date of publication and mention of the grant of the patent: **08.06.2016**
(21) Application number: 10797405.7
(22) Date of filing: 12.07.2010
(51) Int. Cl.: A61F 2/32, A61F 2/34, A61F 2/36, A61B 17/16, A61F 2/46, A61F 2/28, A61F 2/30

(54) **HIP JOINT DEVICE**
HÜFTGELENKVORRICHTUNG
DISPOSITIF D'ARTICULATION DE LA HANCHE

(30) Priority: 10.07.2009 SE 0900981; 10.07.2009 SE 0900957; 10.07.2009 SE 0900959; 10.07.2009 SE 0900960; 10.07.2009 SE 0900962; 10.07.2009 SE 0900963; 10.07.2009 SE 0900965; 10.07.2009 SE 0900966; 10.07.2009 SE 0900968; 10.07.2009 SE 0900969; 10.07.2009 SE 0900970; 10.07.2009 SE 0900972; 10.07.2009 SE 0900973; 10.07.2009 SE 0900974; 10.07.2009 SE 0900976; 10.07.2009 SE 0900978; 10.07.2009 SE 0900958; 30.07.2009 US 229738 P; 30.07.2009 US 229739 P; 30.07.2009 US 229743 P; 30.07.2009 US 229745 P; 30.07.2009 US 229746 P; 30.07.2009 US 229747 P; 30.07.2009 US 229748 P; 30.07.2009 US 229751 P; 30.07.2009 US 229752 P; 30.07.2009 US 229755 P; 30.07.2009 US 229761 P; 30.07.2009 US 229767 P; 30.07.2009 US 229778 P; 30.07.2009 US 229786 P; 30.07.2009 US 229789 P; 30.07.2009 US 229796 P; 30.07.2009 US 229735 P
(43) Date of publication of application: 16.05.2012
(73) Proprietor: Kirk Promotion LTD., 114 79 Stockholm (SE)
(72) Inventor: FORSELL, Peter, CH-6300 Zug (CH)
(86) International application number: PCT/SE2010/050817
(87) International publication number: WO 2011/005198

(56) References cited:
- EP-A1- 0 554 210
- WO-A2-2009/068011
- FR-A1- 2 691 063
- GB-A- 2 007 980
- US-A- 4 044 403
- US-A- 5 263 987
- US-A1- 2003 060 890
- US-A1- 2006 161 261
- US-B1- 6 375 684

## Description

### FIELD OF INVENTION

The present invention relates generally to a medical device for use in a surgical or laparoscopic method of treating hip joint osteoarthritis in a human patient.

### BACKGROUND

Hip Osteoarthritis is a syndrome in which low-grade inflammation results in pain in the hip joints, caused by abnormal wearing of the Cartilage that acts as a cushion inside if the hip joint. This abnormal wearing of the cartilage also results in a decrease of the joints lubricating fluid called Synovial fluid. Hip Osteoarthritis is estimated to affect 80% of all people above 65 years of age, in more or less serious forms.

The present treatments for hip osteoarthritis comprises NSAID drugs, local injections of Hyaluronic acid or Glucocorticoid to help lubricating the hip joint, and replacing parts of the hip joint with a prosthesis through hip joint surgery.

Replacing parts of the hip joint is one of the most common surgeries to date performed at hundreds of thousand of patients in the world annually. The most common method comprises placing a metal prosthesis in Femur and a plastic bowl in Acetabulum. This operation is usually done through a lateral incision in the hip and upper thigh and through Fascia Lata and the lateral muscles of the thigh. To get access to the joint, the supporting Fibrous Capsule attached to Femur and Ilium needs to be penetrated, making it difficult to get a fully functional joint after the surgery. Femur is then cut at the neck with a bone saw and the prosthesis is placed in femur either with bone cement or without. Acetabulum is slightly enlarged using an Acetabular reamer, and the plastic bowl is positioned using screws or bone cement.

The surgery typically requires one week of hospitalization due to the increased risk of infection. The recovery process is on average about 6 weeks, but even after that the patient should not perform any physical activates that places large strain on the joint. FR2691063 (to Perot et al.) discloses a prosthetic hip joint for total hip replacement adapted to be fixated to the femoral bone.

The present invention differs from this prior art in that the prosthetic part has a supporting member connected thereto and in that the prosthetic part can be inserted into a hole in the pelvic bone in an orientation wherein its concave surface is facing towards the femoral head.

### SUMMARY

A medical device for treating hip joint osteoarthritis in a human patient by providing at least one artificial hip joint surface is provided. The hip joint comprises an acetabulum, being a part of the pelvic bone, and a caput femur being a part of the femoral bone, and having a partly spherical form having a largest diameter. The medical device is adapted to be inserted through a hole in the pelvic bone from the opposite side from said acetabulum and to be in contact with the pelvic bone. Furthermore the medical device is adapted to transfer a load from the medical device to the pelvic bone through the contact with the pelvic bone. The use of this medical device enables an operational method that could spare the Fibrous Capsule and reduce the removal of healthy Femur bone. This could also shorten the time for recovery of the patient, and reducing the amount of affected large blood vessels, thus reducing the risk of blood clots.

The inventive medical device is defined in claim 1.

According to one embodiment the medical device has a largest diameter which is adapted to be changed during an operation for treating hip joint osteoarthritis in a human patient by providing at least one artificial hip joint surface. According to one embodiment the medical device is adapted to have a construction, allowing the change in the largest diameter. The construction could comprises at least one slit, at least one elastic member or elastic part or at least two parts adapted to be connected to each other after insertion in a hip joint to form a functional artificial hip joint surface.

According to one embodiment, the at least one artificial hip joint surface comprises an artificial caput femur surface having a largest diameter. According to another embodiment said at least one artificial hip joint surface comprises an artificial acetabulum surface having a largest diameter. According to yet another embodiment the at least one artificial hip joint surface comprises both an artificial caput femur surface and an artificial acetabulum surface.

According to one embodiment the artificial acetabulum surface comprises at least one supporting member. Said at least one supporting member could be adapted to be in connection with the pelvic bone after said medical device has been inserted through said hole in the pelvic bone, and said at least one supporting member is further adapted to carry the load placed on caput femur from the weight of said human patient by the connection with the pelvic bone, after insertion through said hole in the pelvic bone.

The supporting member could comprise at least one element selected from a group consisting of: screws, adhesive, at least one plate, bone cement, or a section of said artificial acetabulum. Said at least one supporting member could be positioned on the abdominal side and/or on the acetabulum side of said pelvic bone for directly or indirectly carrying said load.

According to one embodiment said at least one supporting member is in connection with the bone surrounding said hole, said bone directly or indirectly carrying said load. The supporting member could be adapted to be fixated to the cortex of the pelvic bone or the surface of the pelvic bone for carrying said load.

According to another embodiment said medical device comprises a first and second part, and said second part comprises said supporting member.

### BRIEF DESCRIPTION OF DRAWINGS

The embodiments is now described, by way of example, with reference to the accompanying drawings, in which the figures 30-33 show exemplary embodiments of the claimed invention.
Fig. 1 shows the hip joint in section,
Fig. 2 shows different locations of the incisions made in the human body in the surgical method,
Fig. 3 shows different locations where small incisions can be made in the human body in the laparoscopic method,
Fig. 4 shows the laparoscopic method of operating the hip joint of a human patient,
Fig. 5 shows a lateral view in section of the laparoscopic method,
Fig. 6 shows the hip joint in section when a hole is created in the pelvic bone,
Fig. 7 shows the hip joint in section when a small hole is created in the pelvic bone,
Fig. 8 shows the expandable reamer being used in the surgical or laparoscopic method,
Fig. 9 shows an artificial caput femur surface being larger than equator frustum spherical,
Fig. 10 shows the artificial caput femur surface according to a sixth embodiment,
Fig. 11 shows the artificial caput femur surface according to a sixth embodiment when fixated to the caput femur,
Fig. 12 shows the artificial caput femur surface according to a first embodiment,
Fig. 13 shows the artificial caput femur surface according to a second embodiment,
Fig. 14a-14e shows the artificial caput femur surface according to a third embodiment,
Fig. 15a shows the artificial caput femur surface according to a fourth embodiment,
Fig. 15b shows the artificial caput femur surface according to the fourth embodiment in its folded state,
Fig. 16a shows the artificial caput femur surface according to a seventh embodiment,
Fig. 16b shows the artificial caput femur surface according to the seventh embodiment when assembled,
Fig. 16c shows the artificial caput femur surface according to the seventh embodiment with the connecting members enlarged,
Fig. 17a shows the artificial caput femur surface according to a fifth embodiment,
Fig. 17b shows the artificial caput femur surface according to the fifth embodiment in greater detail,
Fig. 17c shows the artificial caput femur surface according to the fifth embodiment when assembled,
Fig. 18 shows a conceptual view of the function of the expandable caput femur surface,
Fig. 19a shows the step of providing an artificial caput femur surface,
Fig. 19b shows a section of the hip joint after the artificial caput femur surface has been provided,
Fig. 20a shows an expandable artificial caput femur surface, according to the second embodiment, when travelling through a hole in the pelvic bone.
Fig. 20b shows an expandable artificial caput femur surface, according to the second embodiment, when being placed on the caput femur.
Fig. 20c shows an expandable artificial caput femur surface, according to the second embodiment, when placed on the caput femur.
Fig. 21a show the insertion of artificial caput femur surface parts into the hip joint.
Fig. 21b shows the artificial caput femur surface parts after they have been connected inside of the hip joint forming an artificial caput femur surface.
Fig. 21c shows how the form of the artificial caput femur surface parts enables the connection of the artificial caput femur surface parts to form an artificial caput femur surface.
Fig. 21d shows the hip joint in section when a second hole for a camera is provided.
Fig. 22 shows an artificial acetabulum surface when being inserted into a hip joint.
Fig. 23a shows a conceptual view of the function of the expandable acetabulum surface.
Fig. 23b shows the expandable acetabulum surface when positioned.
Fig. 24 shows an artificial acetabulum surface according to a first embodiment.
Fig. 25a shows an artificial acetabulum surface according to a second embodiment.
Fig. 25b shows an artificial acetabulum surface according to the second embodiment in greater detail.
Fig. 25c shows the artificial acetabulum surface when assembled.
Fig. 26a shows an artificial acetabulum surface according to one embodiment.
Fig. 26b shows an artificial acetabulum surface according to one embodiment when assembled.
Fig. 26c shows the connection function of the artificial acetabulum surface according to the embodiment of figs 26a and 26b.
Fig. 27a shows an artificial acetabulum surface according to a fourth embodiment,
Fig. 27b shows the function of the artificial acetabulum surface according to the fourth embodiment,
Fig. 27c shows an artificial acetabulum surface according to a fourth embodiment in its folded state,
Fig. 27d shows an artificial acetabulum surface according to a fourth embodiment in perspective,
Fig. 28a shows an artificial acetabulum surface according to a fifth embodiment,
Fig. 28b shows an artificial acetabulum surface according to the fifth embodiment in its folded state,
Fig. 29a-c shows the closing of a hole in the hip joint using a bone plug,
Fig. 30a,b shows the fixation of a bone plug in the pelvic bone,
Fig. 31 shows a part for closing a hole in the pelvic bone having displaceable supporting members,
Fig. 32a shows a prosthetic part being used to close a hole in the pelvic bone,
Fig. 32b shows how sections of a prosthetic part is used as support against the edges of the hole in the pelvic bone,
Fig. 33 shows the insertion of a prosthetic part in the hole in the pelvic bone,
Fig. 34a shows how screws are being used to fixate a bone plug or a prosthetic part in the hole in the pelvic bone of a human patient,
Fig. 34b shows how a supporting plate is being used to fixate a bone plug or a prosthetic part in the hole in the pelvic bone of a human patient,
Fig. 34c shows two bone plugs or prosthetic parts being fixated using a supporting plate,
Fig. 34d shows a section of the hip joint after two holes in the pelvic bone have been filled with a fluid,
Fig. 35 shows, schematically, the pelvic bone in section.
Fig. 36a shows an injecting member adapted to inject a fluid into an area of the hip joint,
Fig. 36b shows an injecting member adapted to inject a fluid into an area of the hip joint when injecting a fluid,
Fig. 37 shows an injecting member in further detail,
Fig. 38a shows the step of suturing or stapling in the surgical method,
Fig. 38b shows the step of suturing or stapling in the laparoscopic method.

### DETAILED DESCRIPTION OF PREFERRED EMBODIMENTS

Biocompatible material is to be understood as being a material with low level of immune response. Biocompatible materials are sometimes also referred to as biomaterials. Analogous are biocompatible metals, metals with low immune response such as titanium or tantalum. The biocompatible metal could also be a biocompatible alloy comprising at least one biocompatible metal.

Elasticity is to be understood as a materials ability to deform in an elastic way.

Form fitting is to be understood as an element having a part or section which is adapted to enable a mechanical connection of said element to at least one other element using said part or section. Form fitted structure is a structure of an element which enables form fitting.

In the following a detailed description of preferred embodiments will be given. In the drawing figures, like reference numerals designate identical or corresponding elements throughout the several figures. It will be appreciated that these figures are for illustration only and are not in any way restricting the scope. Thus, any references to direction, such as "up" or "down", are only referring to the directions shown in the figures. Also, any dimensions etc. shown in the figures are for illustration purposes.

### REQUIRED STEPS BEFORE THE PLACING OF THE MEDICAL DEVICE

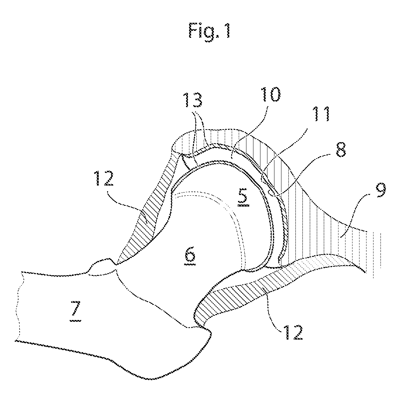

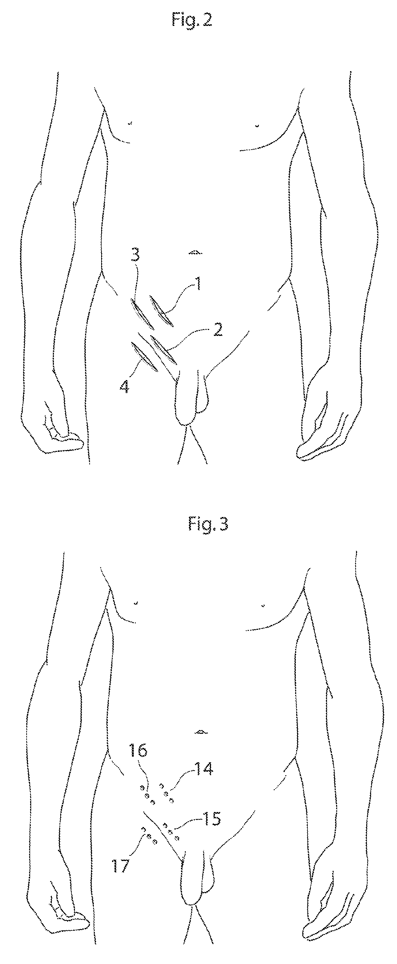

Fig. 1 shows the hip joint of a human patient in section. The hip joint comprises a caput femur 5 which has a partly spherical shape with a diameter, the caput femur 5 is placed at the very top of collum femur 6 which is the top part of the femur bone 7. The caput femur is in connection with the acetabulum 8 which is a bowl shaped part of the pelvic bone 9. Both the caput femur surface 10 and the acetabulum surface 11 is covered with articular cartilage 13 which acts as a cushion in the hip joint. In patients with hip joint osteoarthritis, this articular cartilage 13 is abnormally worn down due to a low grade inflammation. The hip joint is surrounded by the hip joint capsule 12 which provides support for the joint and hinders luxation. After conventional hip joint surgery, penetrating the hip joint capsule 12, the capsule 12 is dramatically weakened due to the limited healing possibilities of its ligament tissue. By performing hip joint surgery without damaging the hip joint capsule 12 the patient can fully recover and place equal amount of strain on an artificial joint as is possible on a natural one. Fig. 2 shows a frontal view of the body of a human patient. A surgical method of operating the hip joint from the opposite side from acetabulum, is according to a first embodiment performed starting with an incision 1 in the abdominal wall of the human patient. The incision 1 passes through the abdominal wall, preferable rectus abdominis and peritoneum in to the abdomen of the human patent. In a second preferred embodiment the incision 2 is conducted in the abdominal wall, preferably through the rectus abdominis and in to the pelvic area, below peritoneum. According to a third embodiment the incision 3 is performed just between Illium of the pelvic bone and the surrounding tissue, an incision 3 which could enable the pelvic bone to be dissected with very little penetration of fascia and muscular tissue. According to a fourth embodiment the incision 4 is made in the inguinal region. In all of the four embodiments the tissue surrounding the pelvic bone 9 (fig.1) in the area opposite to acetabulum 8 (fig.1) is removed or penetrated or divided or moved away which enables the surgeon to reach the pelvic bone 9 (fig.1). It is obvious that the methods described may both be combined or altered reaching the same goal to dissect the pelvic bone on the opposite side of the acetabulum 8 (fig.1).

Fig. 3 shows a frontal view of the body of a human patient. A laparoscopic method of operating the hip joint, from the opposite side from acetabulum, is according to a first embodiment performed starting with making small incisions 14 in the abdominal wall of the human patient. The small incisions enable the surgeon to insert laparoscopic trocars into the abdomen of the human patient. According to the first embodiment the incisions 14 passes through the abdominal wall, preferably rectus abdominis and peritoneum in to the abdomen of the human patent. According to a second preferred embodiment the small incisions 15 is conducted through the abdominal wall, preferably rectus abdominis and in to the pelvic area, below peritoneum. According to a third embodiment the small incisions 16 is performed just between Illium of the pelvic bone and the surrounding tissue, an incision 16 which could enable the pelvic bone to be dissected with very little penetration of fascia and muscular tissue. According to a fourth embodiment the incision 17 is made in the inguinal region. In all of the four embodiments the tissue surrounding the pelvic bone 9 (fig.1) in the area opposite to acetabulum 8 (fig. 1) is removed or penetrated or divided or moved away which enables the surgeon to reach the pelvic bone 9 (fig.1).

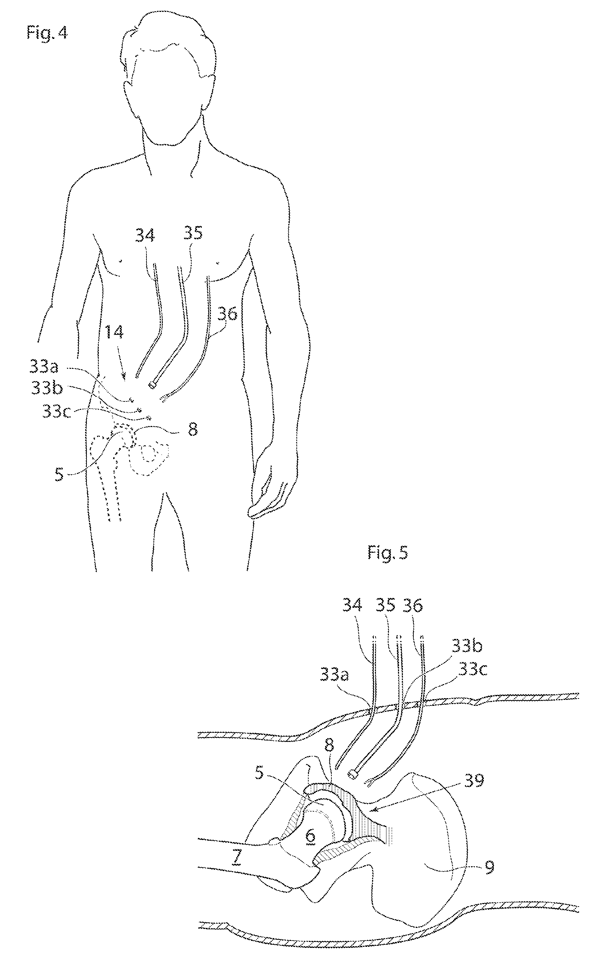

Fig. 4 shows a frontal view of the body of a human patient, illustrating the laparoscopic method of operating the hip joint from the opposite side from acetabulum 8. The hip joint comprising the acetabulum 8 and the caput femur 5. The small incisions 14 in the abdominal wall of the human patient allows the insertion of laparoscopic trocars 33a,b,c into the body of the patients. Whereafter one or more camera 34, a surgical instrument adapted to create a hole in the pelvic bone 35, or instruments 36 for dissecting, introducing, placing, connecting, attaching, creating or filling prosthesis or prosthetic parts, can be inserted into said body through said laparoscopic trocars 33a,b,c.

Fig. 5 shows a lateral view of the body of a human patient, with the hip joint shown in section in further detail. The hip joint comprises a caput femur 5 placed at the very top of collum femur 6 which is the top part of the femur bone 7. The caput femur is in connection with the acetabulum 8 which is a bowl shaped part of the pelvic bone 9. Laparoscopic trocars 33a,b,c is being used to reach the hip joint 39 with one or more camera 34, a surgical instrument adapted to create a hole in the pelvic bone 35, or instruments 36 for dissecting, introducing, placing, connecting, attaching, creating or filling prosthesis or prosthetic parts.

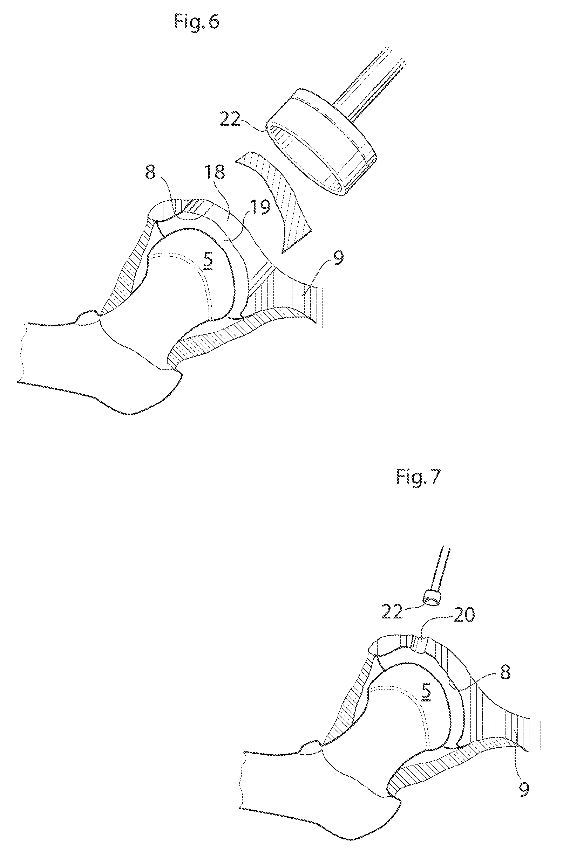

After dissecting the pelvic bone 9 a hole 18 is created in the bone 9, shown in fig. 6. The hole 18 passes through the pelvic bone from the opposite side from acetabulum 8 and into the hip joint 39. The pelvic bone comprises an inner and outer cortex comprising cortical bone. Cortical bone is the outer, more sclerotic bone. The pelvic bone furthermore comprises a bone marrow comprising cancellous bone, which is more fragile.

Fig. 6 shows the hole 18 in the pelvic bone 9 according to a first embodiment, the hole 18 is large which allows prosthesis to pass through said hole 18 in their full functional size. The creation of a hole 18 creates edged of said hole 18 which comprises an inner cortex, on the abdominal side of the pelvic bone, and an outer cortex, on the acetabulum side of the pelvic bone 9.

Fig. 7 shows a second embodiment wherein the hole 20 created in the surgical or laparoscopic method is much smaller, which in turn allows the surgical 35 instrument (fig. 5) creating the hole to be smaller, and thus the incision and dissection performed in the human body could be made smaller.

Before the medical device according to any of the embodiments can be provided, the hip joint surfaces could require preparation. The preparation could comprise reaming the acetabulum 8 and/or the caput femur 5.

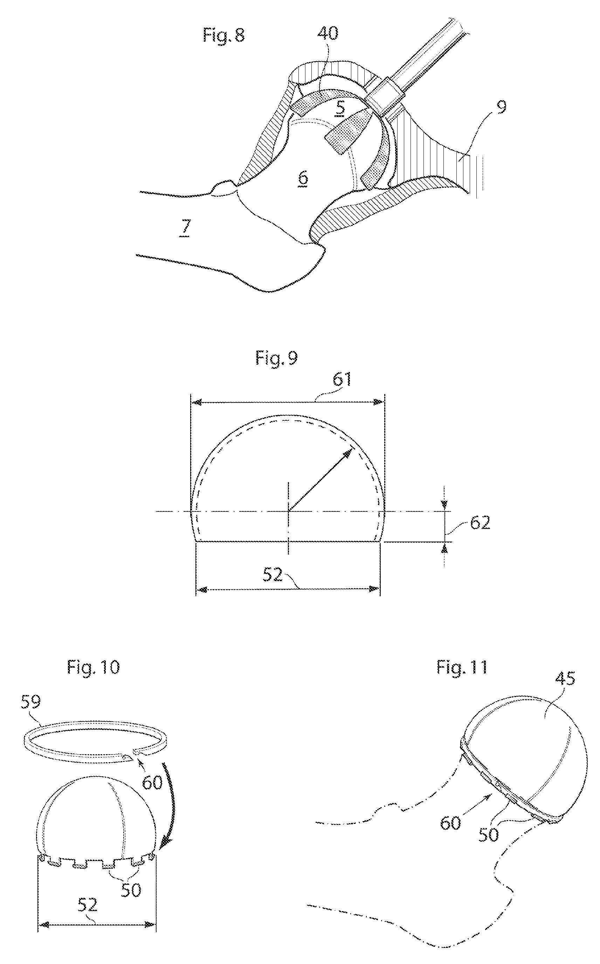

Fig. 8 shows an expandable reamer reaming the acetabulum and/or the caput femur 5. The reamer can be adapted to be operated manually or by means of a rotating, vibrating or oscillating operating device. The reaming prepares the surfaces by removing some of the articular cartilage 13 which covers the contacting surfaces of the acetabulum 8 and the caput femur 5. The removing of the articular cartilage 13 creates room for a medical device comprising at least one hip joint surface, at the same time as it prepares the surfaces for the fixation of the medical device. The expandable reamer comprises multiple reaming blades 40 which in turn comprises abrasive elements or particles adapted to remove material of the hip joint when the expandable reamer is in use.

### THE MEDICAL DEVICE

According to one embodiment, after the preparation of the hip joint surfaces, a medical device comprising an artificial caput femur surface is provided.

Fig. 9 shows an artificial caput femur surface 45 in section having a greatest cross-sectional distance 52 adapted to travel over and beyond the maximum diameter of the caput femur 5. The largest diameter of the caput femur 5 being positioned at a corresponding largest cross sectional distance 61 of the artificial caput femur surface, a second distance 62 is the distance that the artificial caput femur surface 45 travels beyond the maximum diameter of the caput femur 5. Said distance 62 is the beyond part of the artificial caput femur surface and is a part of the mechanical fixation of the artificial caput femur surface 45 to the caput femur 5.

Fig. 10 shows an artificial caput femur surface according to a first embodiment, the artificial caput femur surface 45 is adapted to pass beyond the maximum diameter of the caput femur 5. This enables a mechanical fixation using the form of said artificial caput femur surface 45. In this embodiment the artificial caput femur surface 45 comprises at least one slit 49 adapted to make said artificial caput femur surface 45 flexible for traveling over and beyond the maximum diameter of the caput femur 5. The construction could further be made flexible so that the size of the artificial caput femur surface 45 can vary to become smaller for insertion through a hole 18 in the pelvic bone 9 smaller than the full functional size of the artificial caput femur surface 45. It is also conceivable that the artificial caput femur surface 45 comprises two or more artificial caput femur surface arms 50 which have a cross sectional distance 52 between each other. This cross sectional distance 52 is according to one embodiment shorter than the maximum diameter of the caput femur 5 enabling the mechanical fixation of the artificial caput femur surface 45 by means of said artificial caput femur surface arms 50. For further fixation a band, cord or wire 59 can be placed around the artificial caput femur surface 45 beyond the maximum diameter of the caput femur 5. The band, cord or wire can be mechanically connected using a self locking member 60 for forming a ring-shaped element able to assist in the fixation of the artificial caput femur surface 45 to the caput femur 5.

Fig. 11 shows the artificial caput femur surface 45 when fixated to the caput femur with the supporting band, cord or wire placed around the artificial caput femur surface 45 beyond the maximum diameter of the caput femur 5. The arms may also be adapted to go into the bone of caput femur 5 to lock said artificial caput femur surface 45.

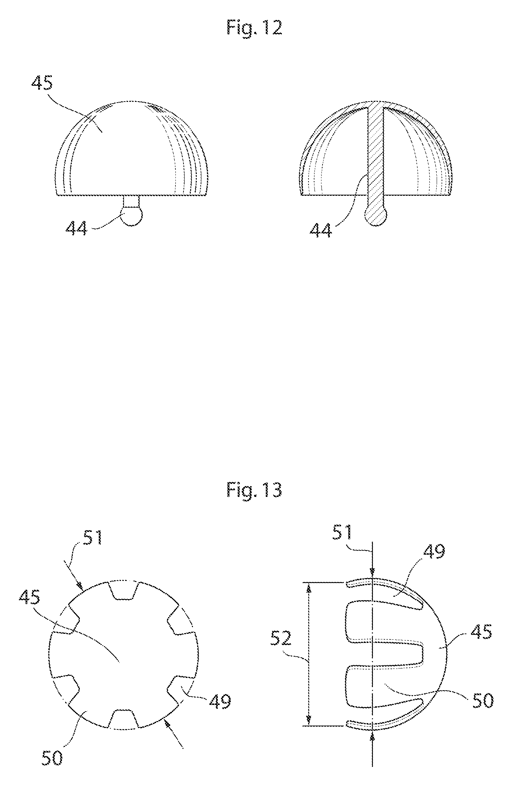

Fig. 12 shows the artificial caput femur surface 45 according to one embodiment. The shaft or screw placed in the middle of the artificial caput femur surface 45 serves as a mechanical attachment 44 penetrating the cortex of the caput femur 5 and fixating the artificial caput femur surface 45 to the caput femur 5. However it is also conceivable that said shaft or screw is assisted or replaced with screws, welding, sprints, band, adhesive or some other mechanical connecting member.

Fig. 13 shows the artificial caput femur surface 45 according to another embodiment, in which said artificial caput femur surface 45 comprises at least one slit 49 enabling the construction of the artificial caput femur surface 45 to be flexible, thus enabling the largest diameter 51 to vary for insertion of said artificial caput femur surface 45 through a hole in the pelvic bone 9 smaller than the full functional size of said artificial caput femur surface 45. According to this embodiment the artificial caput femur surface 45 further comprises artificial caput femur surface arms 50 located on the sides of said at least one slit 49. The caput femur surface arms 50 can be made of a flexible material enabling the insertion through a hole 20 in the pelvic bone 9 smaller than the largest diameter 51 of said artificial caput femur surface 45 when in its full functional size.

According to one embodiment the artificial caput femur surface 45 of said third embodiment could be adapted to pass beyond the maximum diameter of the caput femur 5. This enables a mechanical fixation using the form of said artificial caput femur surface 45. In the embodiment where the artificial caput femur surface 45 travels beyond the maximum diameter of the caput femur 5 the construction can be made flexible so that the size of the artificial caput femur surface 45 can vary to become smaller for insertion through a hole 18 in the pelvic bone smaller than the full functional size of the artificial caput femur surface 45, and have an opening adapter to travel over the caput femur 5 that can be larger that the same opening is in the full functional size of the artificial caput femur surface 45 enabling the artificial caput femur surface 45 to at least partly cover an area beyond the maximum diameter of caput femur 5 from the direction of the acetabulum 8. According to a second embodiment the artificial caput femur surface 45 comprises two or more artificial caput femur surface arms 50 which have a cross sectional distance 52 between each other. This cross sectional distance 52 is according to one embodiment shorter than the maximum diameter of the caput femur 5 enabling the mechanical fixation of the artificial caput femur surface 45 by means of said artificial caput femur surface arms 50.

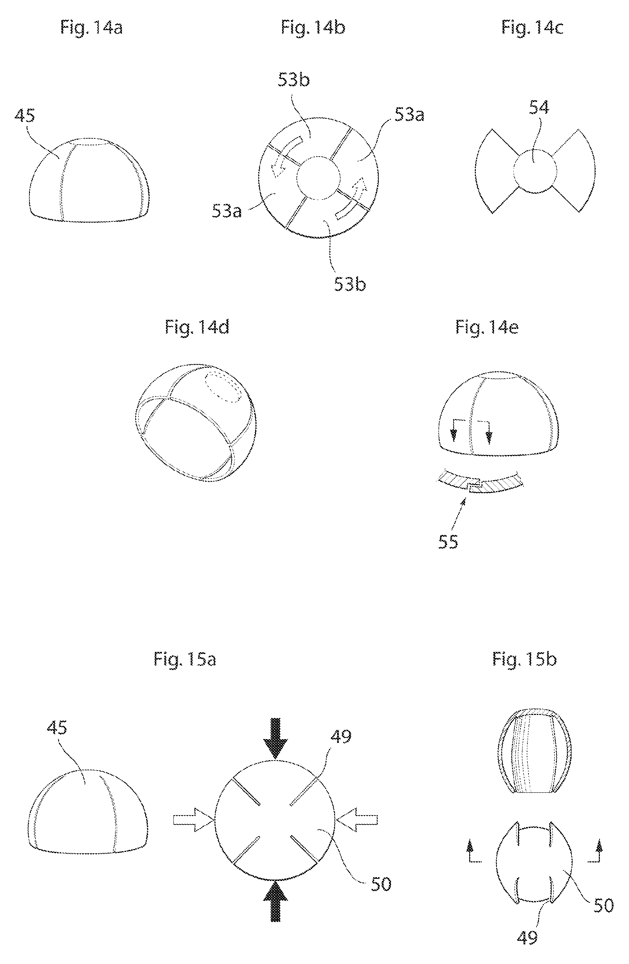

Fig. 14a,b,c,d,e shows the artificial caput femur surface 45 according to a fourth embodiment, in which said artificial caput femur surface 45 comprises a first 53a and a second 53b section, as shown in fig. 14b. The first and second sections are displaceable in relation to each other. According to a first embodiment said first section 53a can be rotated in relation to said second section 53b so that said second section 53b travels underneath said first section 53a to create a displaced artificial caput femur surface 54, as shown in fig. 14c, which is possible to insert into a hip joint of a human patient through a hole 18 being oval, or at least having an area smaller than the cross sectional area of the artificial caput femur surface 45 when in its full functional size 45, as shown in fig. 14a. According to this embodiment the two sections are connected to each other when the artificial caput femur surface 45 is returned to its full functional size using a mechanical form fitting 55, as shown in fig 14e. However it is also conceivable that said connection is assisted or replaced with screws, welding, sprints, band, adhesive or some other mechanical connecting member.

Fig. 15a,b shows the artificial caput femur surface 45 according to another embodiment, in which said artificial caput femur surface 45 comprises four slits. The artificial caput femur surface 45 is flexible in its construction allowing the four artificial caput femur arms 50 to be folded towards the center axis of the artificial caput femur surface 45 thus allowing the artificial caput femur surface 45 to be inserted into a hip joint through a hole smaller than the full functional size of the artificial caput femur surface 45. The artificial caput femur surface 45 according to this embodiment can be constructed to go beyond the maximum diameter of the caput femur 5, in which case the construction with the slits 49 allows the artificial caput femur surface 45 to change to both a smaller and a larger size than said full functional size.

Fig. 15b shows the artificial caput femur surface 45 in section when said artificial caput femur surface arms 50 are folded for insertion through a hole 18 with an area smaller than the largest area of the artificial caput femur surface 45 when in its full functional size.

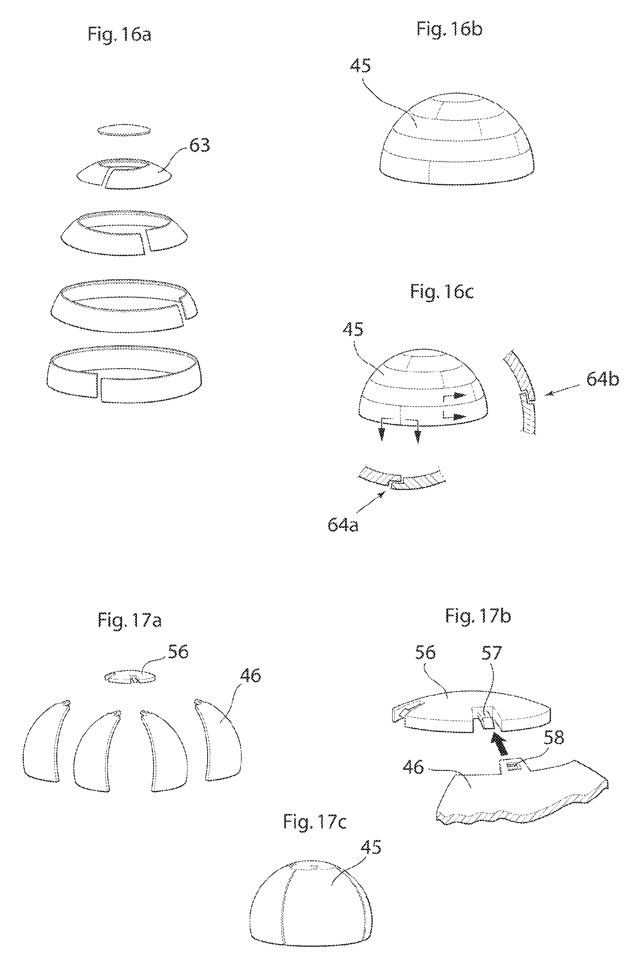

Fig 16a shows the artificial caput femur surface 45 according to a sixth embodiment, in which said artificial caput femur surface 45 comprises multiple ring-shaped artificial caput femur surface parts 63. Said multiple ring-shaped artificial caput femur surface parts 63 are adapted to be connected to each other to form an artificial caput femur surface 45, shown in fig. 16b. According to one embodiment said artificial caput femur surface parts 63 are adapted to be connected to each other using mechanical connecting members 64a,b. In Fig. 16c, 64a shows how an individual ring-shaped artificial caput femur surface part 63 can be connected to itself to form a continuous ring shape. 64b shows how an individual ring-shaped artificial caput femur surface part 63 connects to other ring-shaped artificial caput femur surface parts 63 to form an artificial caput femur surface 45. The artificial caput femur surface 45 according to this embodiment can further be adapted to go beyond the maximum diameter of the caput femur 5.

Fig. 17a,b,c shows the artificial caput femur surface 45 according to a sixth embodiment, in which said artificial caput femur surface 45 comprises multiple artificial caput femur surface parts 46. Said multiple artificial caput femur surface parts 46 are adapted to be connected to an interconnecting artificial caput femur surface part 56 after insertion into a hip joint. The interconnecting artificial caput femur surface part 56, which serves as a base part, comprises self locking connecting members 57, shown in fig. 17b, that fits with corresponding self locking members 58 of the artificial caput femur surface parts 46. The artificial caput femur surface parts 46 create an artificial caput femur surface 45 when connected to each other, shown in fig. 17c. The self locking members 57, 58 can be assisted or replaced with screws, welding, sprints, band, adhesive or some other mechanical connecting member. The artificial caput femur surface 45 according to this embodiment can further be adapted to go beyond the maximum diameter of the caput femur 5.

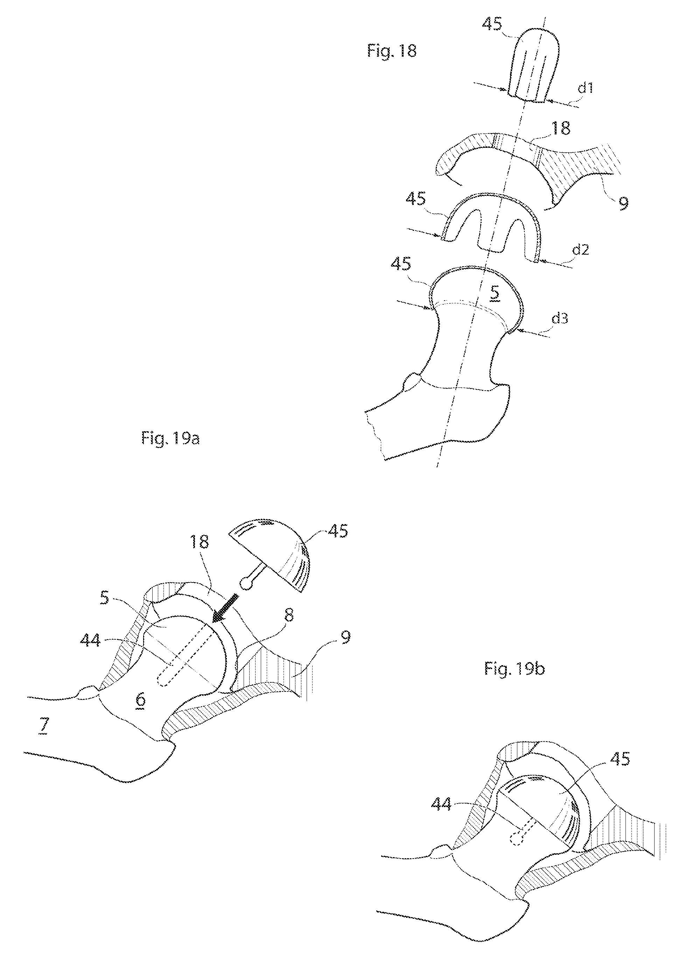

Fig. 18 shows a conceptual way wherein the artificial caput femur surface 45 has a diameter or cross-sectional distance d1 small enough to enable said artificial caput femur surface 45 to travel through a hole 20 in the pelvic bone 9. After the artificial caput femur surface 45 has traveled through the hole 20 in the pelvic bone 9 the artificial caput femur surface 45 is expanded such that the diameter or cross-sectional distance d2 is large enough to travel over the caput femur 5. Finally the artificial caput femur surface 45 is positioned on the caput femur 5, in this state the diameter or cross-sectional distance is smaller than the largest diameter of the caput femur 5 which mechanically attaches the artificial caput femur surface 45 to the caput femur 5. d3 is the normal sate cross sectional distance of the medical device, i.e. the cross sectional distance that the medical device has when the medical device is in its functional position. This figure may also in an alternative embodiment show the artificial acetabulum surface mounted onto caput femur or an artificial replacement therefore with the same locking principle.

Fig 19a,b shows the hip joint in section with the caput femur 5 placed at the very top of collum femur 6, which is the top part of the femur bone 7. The caput femur is in connection with the acetabulum 8, which is a bowl shaped part of the pelvic bone 9. According to a first embodiment the hole 18 created in the pelvic bone 9 from the opposite side from acetabulum 8, is larger than said artificial caput femur surface 45, enabling the insertion of said artificial caput femur surface 45 in its full functional size. Said insertion of said artificial caput femur surface 45 could be performed as a step of the surgical method, as well as a step of the laparoscopic method. After the insertion, the artificial caput femur surface 45 is attached to the caput femur 5, the attaching is performed by means of a mechanical attachment 44 comprising a shaft or screw penetrating the cortex. It is however also conceivable that the mechanical attachment 44 is assisted or replaced by bone cement or adhesive placed between caput femur 5 and the artificial caput femur surface 45, or in connection with said shaft or screw 44. Alternative ways of attaching the artificial caput femur surface 45 includes: at least one screw, at least one pin, at least one portion of at least one of the parts adapted to be introduced into the other part, the parts being adapted to be sliding into the other part, form fitting, welding, adhesive, pin, wire, a ball mounted into a bowl being portions of said parts, a male portion of one part mounted into a female portion of the other part, a key introduced into a lock being portions of said parts, band, or other mechanical connecting members.Fig. 19b shows the hip joint in section with the artificial caput femur surface 45 attached to the caput femur 5.

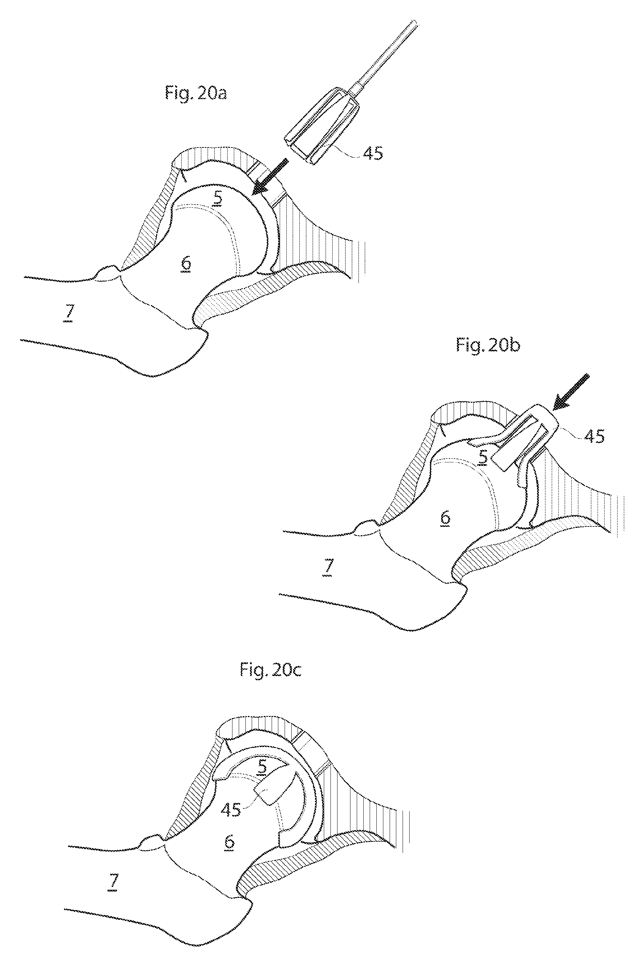

Fig. 20a shows how an expandable artificial caput femur surface 45 is being inserted through a hole 18 in the pelvic bone 9.

Fig. 20b shows how an expandable artificial caput femur surface 45 travels through the hole 18 in the pelvic bone 9 and travels over caput femur 5.

Fig. 20c shows an expandable artificial caput femur surface 45 is after it has been placed on said caput femur 5.

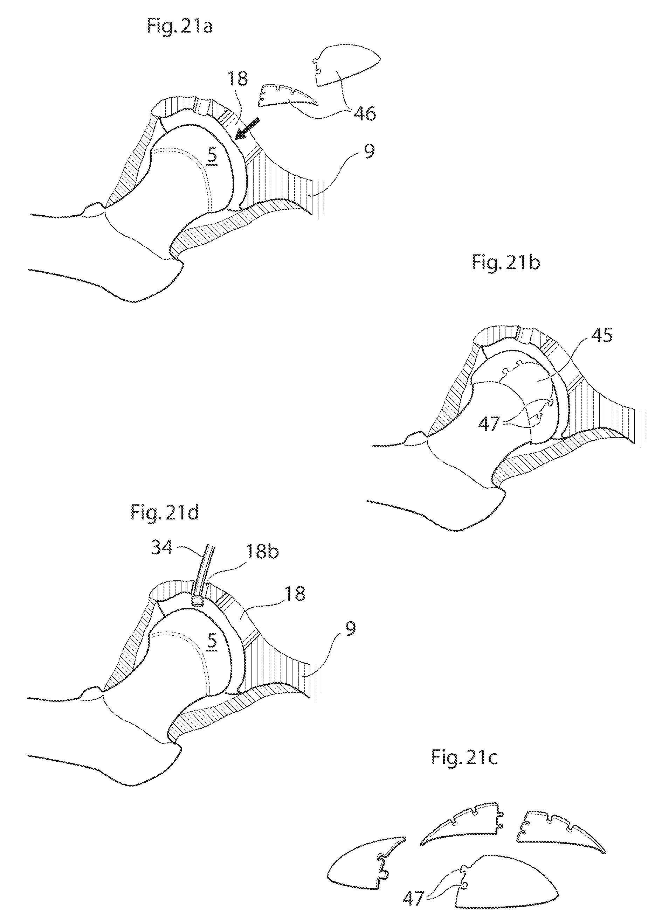

Fig. 21a shows the hip joint in section according to a second embodiment in which the hole 18 in the pelvic bone 9 is smaller than the artificial caput femur surface 45 in its full functional size. According to this embodiment the artificial caput femur surface 45 is introduced into said hip joint through the hole 18 in the pelvic bone 9 form the opposite side from acetabulum 8. The artificial caput femur surface parts 46 are connected to each other after insertion into said hip joint to form the artificial caput femur surface 45.

Fig. 21b shows the hip joint in section when the artificial caput femur surface parts 46 are connected to each other using form fitting 47, however it is conceivable that the form fitting is assisted or replaced with adhesive or bone cement. After the artificial caput femur surface parts 46 have been introduced and connected in the hip joint, they are mechanically fixated to the caput femur 5, the mechanical fixation could be done by means of: at least one screw, at least one pin, at least one portion of at least one of the parts adapted to be introduced into the other part, the parts being adapted to be sliding into the other part, form fitting, welding, adhesive, pin, wire, a ball mounted into a bowl being portions of said parts, a male portion of one part mounted into a female portion of the other part, a key introduced into a lock being portions of said parts, band, or other mechanical connecting members.

Fig. 21c shows the artificial caput femur surface parts 46 with the parts supplying the form fitting 47.

Fig. 21d shows the hip joint in section wherein a second hole 18b in the pelvic bone 9 enables the surgeon to place a camera 34 into the hip joint, preferably used in the laparoscopic method.

According to one embodiment the medical device comprises an artificial acetabulum surface 65. In the embodiments where the medical device comprises an artificial caput femur surface 45 and an artificial acetabulum surface 65, the artificial acetabulum surface is provided after the artificial caput femur surface.

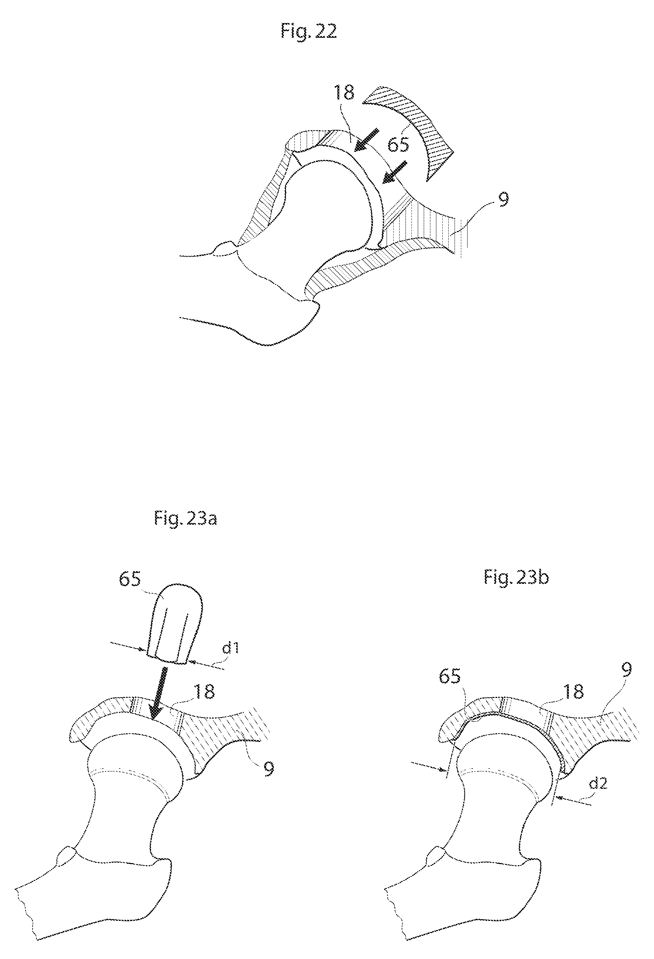

Fig. 22 shows an artificial acetabulum surface 65 in its full functional size, as it is being inserted through a hole 18 in the pelvic bone 9. The hole being large enough to allow the artificial acetabulum surface to pass through the hole.

Fig. 23a shows an artificial acetabulum surface in a conceptual way, wherein the artificial acetabulum surface 65 has a diameter or cross-sectional distance d1 small enough to enable said artificial acetabulum surface 65 to travel through a hole 18 in the pelvic bone 9. After the artificial acetabulum surface 65 has traveled through the hole 18 in the pelvic bone 9, the artificial acetabulum surface is expanded such that the diameter or cross-sectional distance d2 is large enough to hinder the artificial acetabulum surface 65 from traveling through the hole 18 in the pelvic bone 9.

Fig. 23b shows the artificial acetabulum surface 65 when positioned in the acetabulum 8.

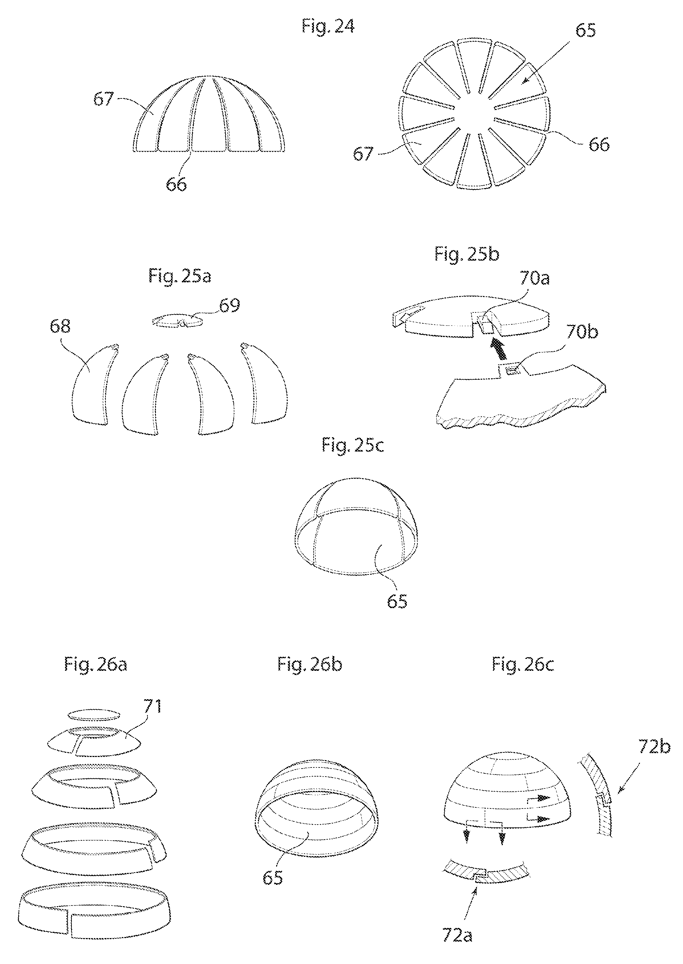

Fig. 24 shows an artificial acetabulum surface 65 according to a second embodiment in which the artificial acetabulum surface 65 comprises at least one slit 66 enabling the artificial acetabulum surface 65 to vary in size for insertion through a hole 18 in the pelvic bone 9 smaller than the full functional size of the artificial acetabulum surface 65. The slits are placed between one or more artificial acetabulum surface arms 67 which are flexible by means of the material or by means of a joint affecting said artificial acetabulum surface arms 67.

Fig. 25a,b,c shows an artificial acetabulum surface 65 according to a second embodiment in which the artificial acetabulum surface 65 comprises multiple artificial acetabulum surface parts 68. Said multiple artificial acetabulum surface parts 68 are adapted to be connected to an interconnecting artificial acetabulum surface part 69 after insertion into a hip joint. The interconnecting artificial caput femur surface part 69 comprises self locking connecting members 70a, shown in fig. 25b, that fits with corresponding self locking members 70b of the artificial acetabulum surface parts 68. The artificial acetabulum surface parts 68 create an artificial acetabulum surface 65 when connected to each other, shown in fig. 25c. The self locking members 70a,b can be assisted or replaced with at least one screw, at least one pin, at least one portion of at least one of the parts adapted to be introduced into the other part, the parts being adapted to be sliding into the other part, form fitting, welding, adhesive, pin, wire, a ball mounted into a bowl being portions of said parts, a male portion of one part mounted into a female portion of the other part, a key introduced into a lock being portions of said parts, band, or other mechanical connecting members.

Fig. 26a,b,c shows an artificial acetabulum surface 65 according to a third embodiment in which the artificial acetabulum surface 65 comprises multiple ring-shaped artificial acetabulum surface parts 71. Said multiple ring-shaped artificial acetabulum surface parts 71 are adapted to be connected to each other to form an artificial acetabulum surface 65 after insertion in a hip joint. According to one embodiment said artificial acetabulum surface parts 71 are adapted to be connected to each other using mechanical connecting members 72a,b.

Fig. 26c shows how an individual ring-shaped artificial acetabulum surface part 71 can be connected to itself using the mechanical connecting member 72a to form a continuous ring shape. Further 37c shows how an individual ring-shaped artificial acetabulum surface part 71 connects to other ring-shaped artificial acetabulum surface parts 71 using the mechanical connecting member 72b to form an artificial acetabulum surface 65.

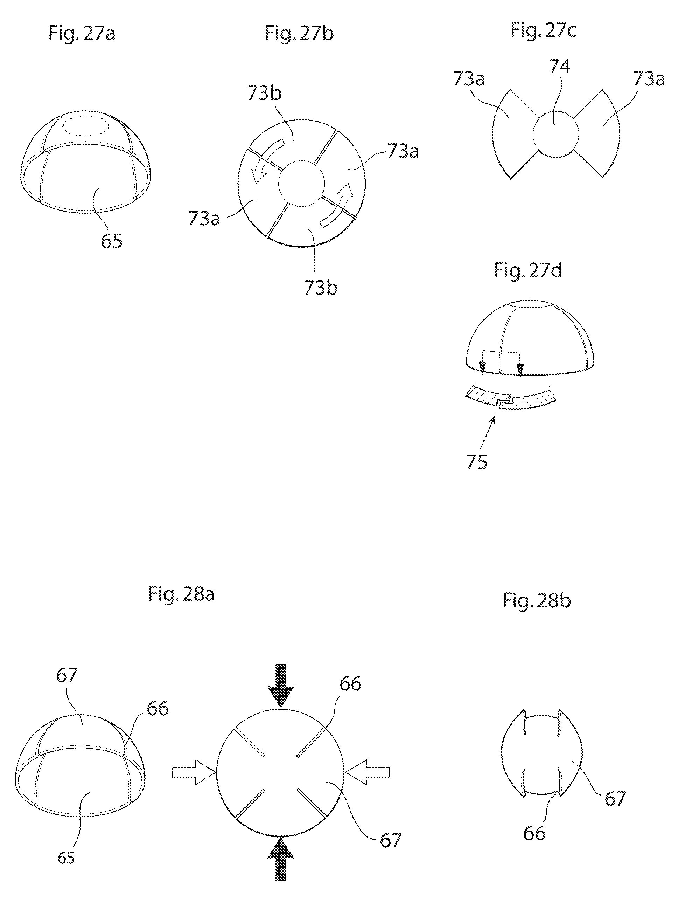

Fig. 27a,b,c,d shows an artificial acetabulum surface 65 according to a fourth embodiment in which the artificial acetabulum surface 65 comprises a first 73a and a second 73b section, shown in fig. 27b. The first and second sections are displaceable in relation to each other. According to one embodiment said first section 73a can be rotated in relation to said second section 73b so that said second section 73b travels underneath said first section 73a to create a displaced artificial acetabulum surface 74, as shown in fig. 27c, which is possible to insert into a hip joint of a human patient through a hole being oval, or at least having an area smaller than the cross sectional area of the artificial acetabulum surface 65 when in its full functional size 65. According to this embodiment the two sections 73a,b are connected to each other when the artificial acetabulum surface is returned to its full functional size using a mechanical form fitting 75, as shown in fig. 27d. However it is also conceivable that said connection is assisted or replaced with at least one screw, at least one pin, at least one portion of at least one of the parts adapted to be introduced into the other part, the parts being adapted to be sliding into the other part, form fitting, welding, adhesive, pin, wire, a ball mounted into a bowl being portions of said parts, a male portion of one part mounted into a female portion of the other part, a key introduced into a lock being portions of said parts, band, or other mechanical connecting members.

Fig. 28a shows an artificial acetabulum surface 65 according to a fifth embodiment in which the artificial acetabulum surface 65 comprises four slits 66. The artificial acetabulum surface 65 is flexible in its construction allowing the four artificial acetabulum arms 67 to be folded towards the center axis of the artificial acetabulum surface 65 thus allowing the artificial acetabulum surface to be inserted into a hip joint through a hole smaller than the full functional size of the artificial acetabulum surface 65.

Fig. 28b shows the artificial acetabulum surface 65 according to the fifth embodiment in its folded state.

After the medical device, comprising at least one hip joint surface, has been provided through a hole 18 in the pelvic bone 9, in accordance with any of the embodiment above, said hole 18 needs to be closed. The hole can be closed using the medical device, however it is also conceivable that closing of the hole is performed with assistance of an additional prosthetic part, or a piece of human bone. In the embodiments where the closing is assisted by a piece of human bone, that piece could simply be the piece drilled from the pelvic bone or a modified piece from the pelvic bone e.g. equipped with additional supporting members.

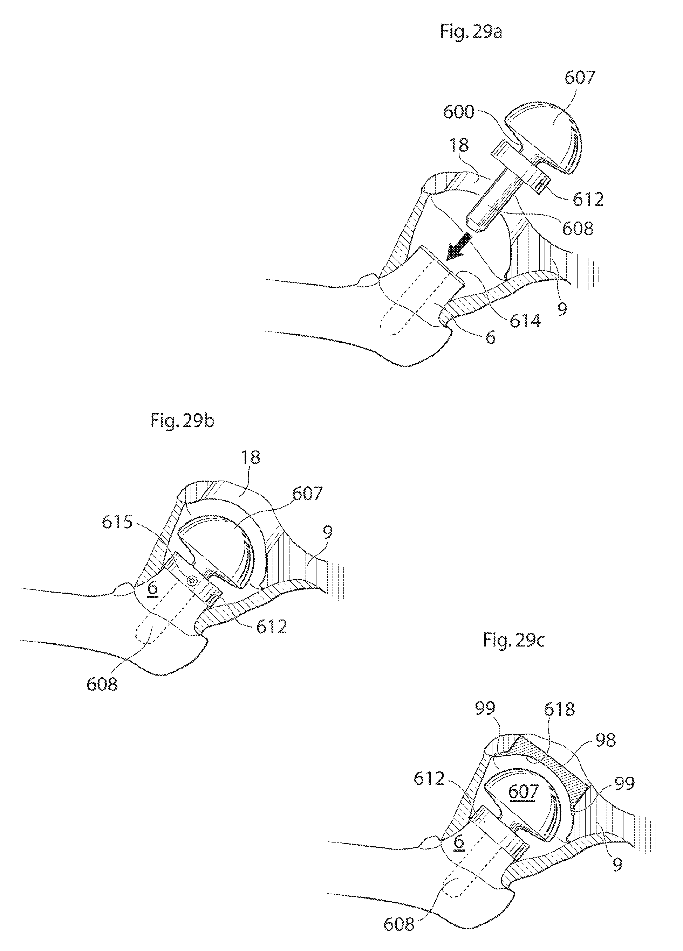

Fig. 29a shows an embodiment where a solid medical device 600 is fixated to the collum femur 6, introduced through a hole 18 in the pelvic bone 9. The stabilizing member 612 is adapted to stabilize the medical device 600 from the outside of the collum femur 6 substantially perpendicular to the longitudinal extension of the collum femur 6, and from the acetabulum side, substantially in line with the longitudinal extension of the collum femur 6 through the stabilizing member being placed in contact with the outside of the collum femur 6 and the surface of the section 610 in the collum femur 6. The stabilizing member is fixated to the outside of the collum femur and/or to the surface of the section in the collum femur by means of the adhesive 614. However the adhesive 614 could be replaced or assisted by bone cement or a mechanical fixation element 615. The medical device 600 has a smallest passable area being an area of a hole 18 through which the medical device 600 can pass. To enable the solid medical device 607 to pass through said hole in the pelvic bone the solid medical device 600 is smaller than the caput femur, i.e. the smallest passable area of the medical device 600, being an area of a hole through which said medical device 600 can pass is smaller than the smallest passable area of the caput femur 5, being an area of a hole through which said caput femur 5 can pass.

Fig. 29b shows the hip joint in section when the medical device 600 is positioned on the collum femur 6. The stabilizing member 612 is here fixated to the collum femur by means of adhesive 614 and a mechanical fixation element 615.

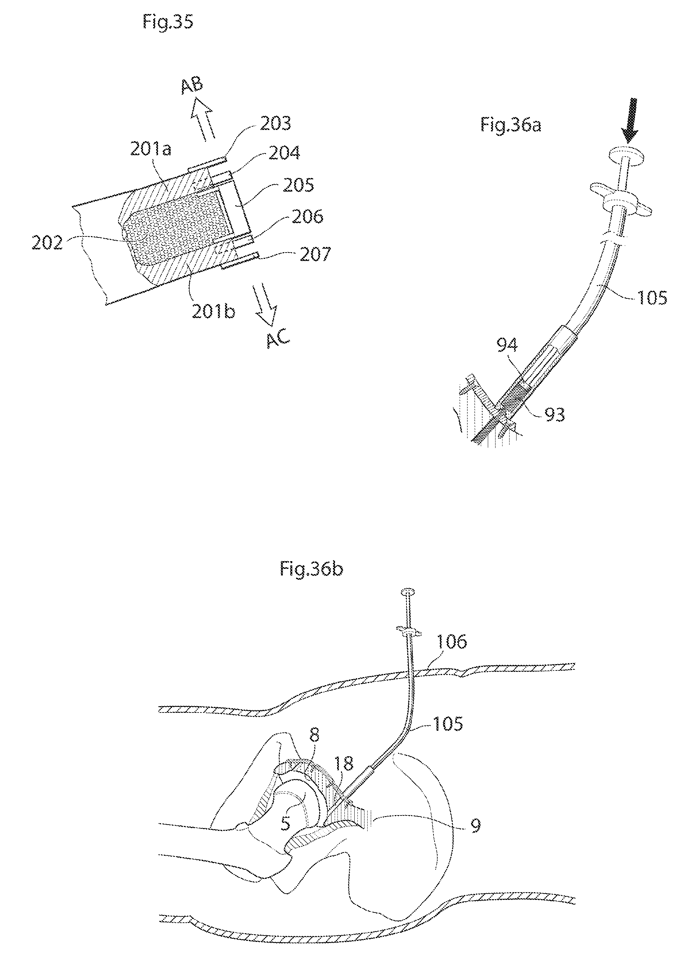

Fig. 29c shows the hip joint in section when the medical device 600 is positioned on the collum femur 6. The stabilizing member 612 is here fixated to the collum femur by means of adhesive 614. An prosthetic part 98 comprising an artificial acetabulum surface 618 has been positioned in the hole 18 in the pelvic bone 9. The artificial acetabulum surface 618 is adapted to be in direct of indirect connection with the artificial caput femur surface 607. In embodiments where the artificial acetabulum surface 618 is adapted to be in indirect connection with the artificial caput femur surface 607 a lubricating fluid or a lubricating material (not shown) can be placed between said artificial acetabulum surface 618 and said artificial caput femur surface 607. The prosthetic part is adapted to carry the load placed on the artificial acetabulum surface 618 from weight of the human patient through the contact with the artificial caput femur surface 607 by means of the supporting members 99 in connection with the pelvic bone. The prosthetic part 98 can further be fixated to the pelvic bone 9 by means of bone cement, adhesive, screws, form fitting, welding, sprints, band or some other mechanical connecting member. According to this embodiment the supporting members 99 are positioned on the acetabulum side of the pelvic bone 9, however it is also conceivable that the supporting members 99 are positioned on the abdominal side of the pelvic bone 9 or according to fig. 35.

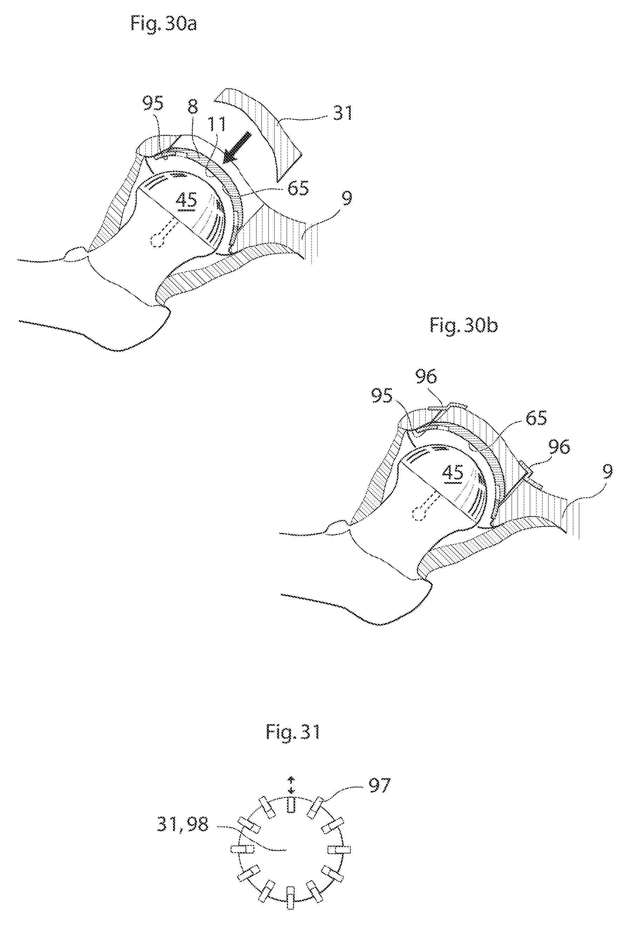

Fig. 30a shows the hip joint of a human patient in section, wherein a bone plug 31 is placed in the hole 18 in the pelvic bone 9 to close said hole 18. According to a first embodiment the medical device comprises supporting members 95 which carries the load placed on the acetabulum 8 from weight of the human patient through the contact with the caput femur 5. Said supporting members can be adapted to be displaceable 97 supporting members. The bone plug 31 can be attached to the artificial acetabulum surface 11 and/or the pelvic bone 9 by means of at least one screw, at least one pin, at least one portion of at least one of the parts adapted to be introduced into the other part, the parts being adapted to be sliding into the other part, form fitting, welding, adhesive, pin, wire, a ball mounted into a bowl being portions of said parts, a male portion of one part mounted into a female portion of the other part, a key introduced into a lock being portions of said parts, band, or other mechanical connecting members.

The connection between the medical device and the pelvic bone 9 can be direct or indirect. In the embodiment where the contact between the medical device and the pelvic bone 9 is indirect, a material can be provided between said medical device and said pelvic bone. The material could comprise bone cement, an at least partly elastic material, glue, adhesive, antibiotic, biocompatible plastic material, biocompatible ceramics and/or biocompatible metal.

Fig 30b shows the hip joint of a human patient in section wherein the bone plug 31 placed in the hole 18 in the pelvic bone 9 is further supported by supporting members 96 placed between the bone plug 31 and the pelvic bone 9 on the opposite side from acetabulum 8 using at lest one of: at least one screw, at least one pin, at least one portion of at least one of the parts adapted to be introduced into the other part, the parts being adapted to be sliding into the other part, form fitting, welding, adhesive, pin, wire, a ball mounted into a bowl being portions of said parts, a male portion of one part mounted into a female portion of the other part, a key introduced into a lock being portions of said parts, band, or other mechanical connecting members.

Fig. 31 shows a bone plug 31 or a prosthetic part 98 comprising several displaceable supporting members adapted to carry the load placed on the acetabulum 8 from weight of the human patient through the contact with the caput femur 5. The displaceable parts 97 are displaced into a corresponding part in or at the edge of the hole 18 in the pelvic bone 9.

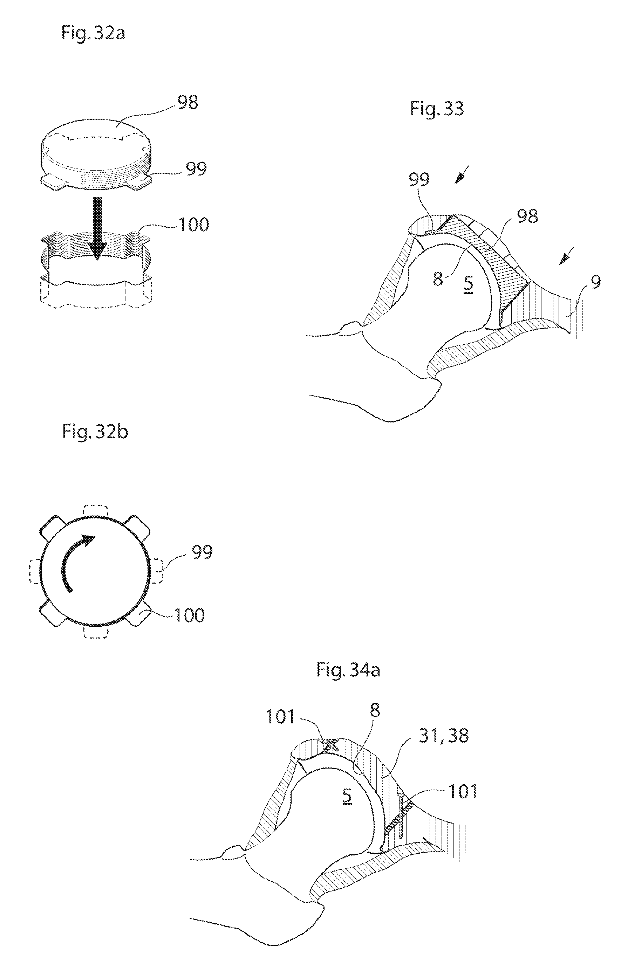

Fig 32a shows an embodiment wherein the closing of the hole 18 in the pelvic bone is performed by means of a prosthetic part 98. Fig. 56a shows the prosthetic part 98 being inserted into a hole 18 in the pelvic bone 9 from the opposite side from acetabulum 8. According to one embodiment the prosthetic part 98 comprises supporting members 99 adapted to correspond with sections 100 of the hole 18 in the pelvic bone 9. After the prosthetic part 98 has been inserted into said hole 18 in the pelvic bone 9 it is rotated so that the supporting members 99 comes in contact with the pelvic bone 9 and can carry the load placed on the acetabulum 8 from weight of the human patient through the contact with the caput femur 5. Said prosthetic part 98 could also be adapted to serve as artificial acetabulum surface 65 according to any of the above mentioned embodiments.

Fig. 32b shows the prosthetic part 98 when rotated to carry the load placed on the acetabulum 8 from weight of the human patient through the contact with the caput femur 5.

This supporting means could be constructed in many different ways and this should be seen as examples.

Fig. 33 shows the hip joint of a human patient in section wherein the prosthetic part 98 closes the hole 18 in the pelvic bone 9 and carries the load placed on the acetabulum 8 from weight of the human patient through the contact with the caput femur 5 by means of the supporting members 99. The prosthetic part 98 can further be fixated to the pelvic bone 9 by means of at least one screw, at least one pin, at least one portion of at least one of the parts adapted to be introduced into the other part, the parts being adapted to be sliding into the other part, form fitting, welding, adhesive, pin, wire, a ball mounted into a bowl being portions of said parts, a male portion of one part mounted into a female portion of the other part, a key introduced into a lock being portions of said parts, band, or other mechanical connecting members.

Fig. 34a shows the hip joint of a human patient in section wherein bone plug 31 or prosthetic part 98 is attached to the pelvic bone 9 by means of screws 101 placed from the opposite side from acetabulum 8. The screws 101 are possible to place in different angles depending on reach or need for support. This construction may be performed in many different ways, for example using a plate mounted to the pelvic bone.

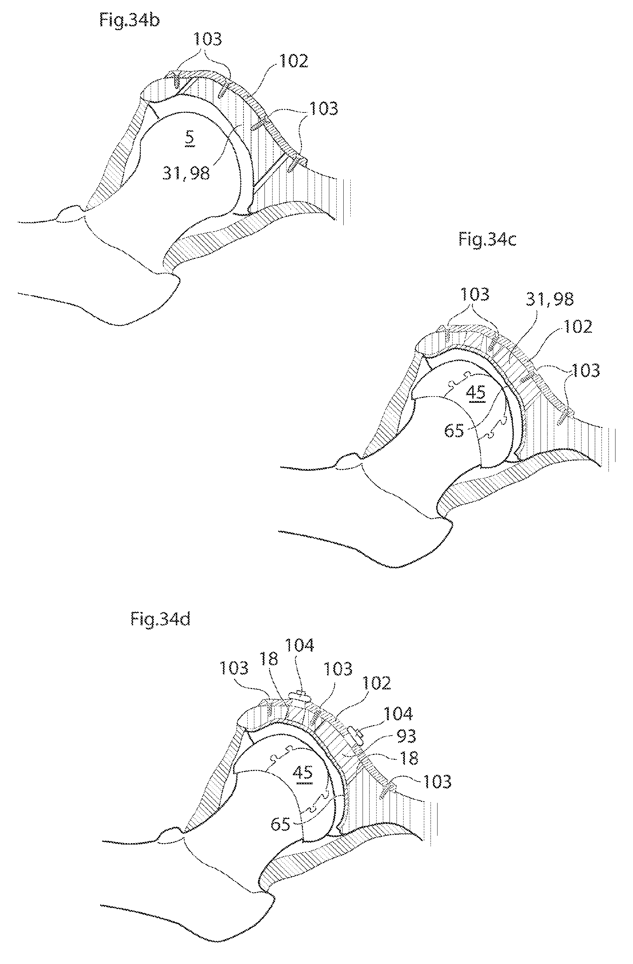

Fig 34b shows the hip joint of a human patient in section wherein bone plug 31 or prosthetic part 98 is attached to the pelvic bone 9 by means of a plate 102 at least partly covering said bone plug 31 or prosthetic part 98. According to a first embodiment the plate 102 is attached to the pelvic bone 9 by means of screws 103 placed from the opposite side from acetabulum 8. However it is also conceivable that said screws 103 can be replaced or assisted by bone cement, adhesive, form fitting, welding, sprints, band or some other mechanical connecting member.

Fig. 34c shows the hip joint of a human patient in section wherein two bone plugs 31 or prosthetic parts 98 are attached to the pelvic bone 9 by means of a plate 102 at least partly covering said bone plugs 31 or prosthetic parts 98. According to a first embodiment the plate 102 is attached to the pelvic bone 9 by means of screws 103 placed from the opposite side from acetabulum 8. However it is also conceivable that said screws 103 can be replaced or assisted by bone cement, adhesive, form fitting, welding, sprints, band or some other mechanical connecting member.

Fig 34c also shows the provided artificial acetabulum surface 65 and the provided caput femur surface. The members for fixating and covering the hole in the pelvic bone, together with the artificial acetabulum surface and the artificial caput femur surface constitute the medical device.

Fig. 34d shows the hip joint of a human patient in section wherein two holes 18 in the pelvic bone has been covered by means of a fluid injected into said holes 18, through sealing members 104, said fluid 93 being adapted to harden. Further more a plate 102 has been provided at least partly covering said holes 18. According to a first embodiment the plate 102 is attached to the pelvic bone 9 by means of screws 103 placed from the opposite side from acetabulum 8. However it is also conceivable that said screws 103 can be replaced or assisted by bone cement, adhesive, form fitting, welding, sprints, band or some other mechanical connecting member. Fig 34d also shows the provided artificial acetabulum surface 65, and the provided artificial caput femur surface 45.

Fig. 35 is a schematic figure of the pelvic bone in section, describing in further detail the supporting members shown in for example fig. 35. The pelvic bone comprises an inner cortex 201a placed on the abdominal side of the pelvic bone AB, and an outer cortex 201b placed on the acetabulum side of the pelvic bone AC. The inner and outer cortex 201a,b comprises cortical bone, which is a more dense sclerotic bone. The pelvic bone further comprises cancellous bone 202, placed in the bone marrow between said inner cortex 201a and said outer cortex 201b. The supporting members of the medical device according to any of the embodiments above can be adapted to be in contact with the outside of the inner cortex 201 a as supporting member 203, or be placed inside of the inner cortex 201a as supporting member 204, which enables the supporting member to carry loads in the direction of the abdomen AB as well as in the direction of the acetabulum AC. It is furthermore conceivable that the supporting member is placed in the middle of the inner cortex 201a and the outer cortex 201b, in the cancellous bone, as supporting member 205, in which case the supporting member could be in contact with the inner cortex 201 a, on the inside thereof, and the outer cortex 201b, on the inside thereof, which enables the supporting member to carry loads in the direction of the abdomen AB as well as in the direction of the acetabulum AC. Further, the supporting members can be adapted to be in contact with the outside of the outer cortex 201b as supporting member 207, or be placed inside of the outer cortex 201b as supporting member 206, which enables the supporting member to carry loads in the direction of the abdomen AB as well as in the direction of the acetabulum AC.

Fig. 36a shows an injecting member 105 for injecting a fluid adapted to harden 93, preferably bone cement or adhesive to be used as support in the closing of the hole 18 in the pelvic bone 9. The injecting member 105 comprises a piston 94 that pushes said fluid 93 in to the area where it is wanted.

Fig. 36b shows the injecting member 105 as it is inserted through the skin 106 of a human patient in the surgical or laparoscopic method, and is further placed in connection with the hip joint through the hole 18 in the pelvic bone 9. The injecting member 105 is adapted to inject a fluid 93 adapted to harden.

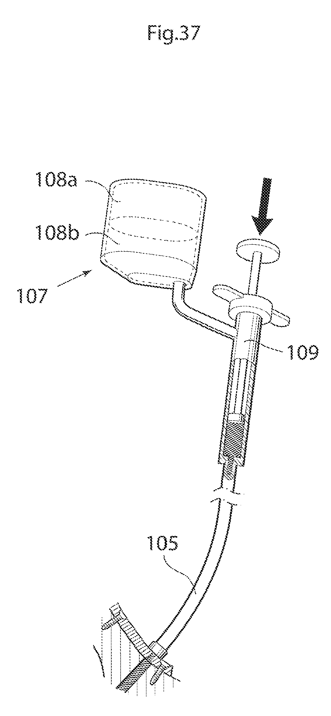

Fig. 37 shows the injecting member 105 according to any of the embodiments above, adapted to inject fluid 93 into a mould 81, a sealed area 87 or a connecting area between the pelvic bone 9 and a prosthetic part, the pelvic bone 9 and a bone plug 31 or the caput femur 5 and a prosthetic part. Said injecting member comprises a container 107 adapted to hold a fluid for injection. According to a first embodiment said container comprises two compartments 108a,b adapted to hold two different fluids, said fluids being adapted to harden when mixed. In the embodiment when the container 107 is adapted to hold two fluids, it is conceivable that the injecting member 105 further comprises a mixing member 109 wherein said two fluids are being mixed before injection. According to a second embodiment (not shown) said container 107 is adapted to keep said fluid sterile. According to a third embodiment (not shown) said container 107 is adapted to keep said fluid cold and according to a fourth embodiment (not shown) said container 107 is adapted to keep said fluid in a dark environment. Furthermore a combination of the above mentioned embodiments is conceivable.

After the step of closing the hole in the pelvic bone of the human patient is concluded the medical device has been provided and all instruments are retracted. The final step of a surgical or laparoscopic method comprises suturing or stapling the affected tissue and finally suturing or stapling the skin of the human patient.

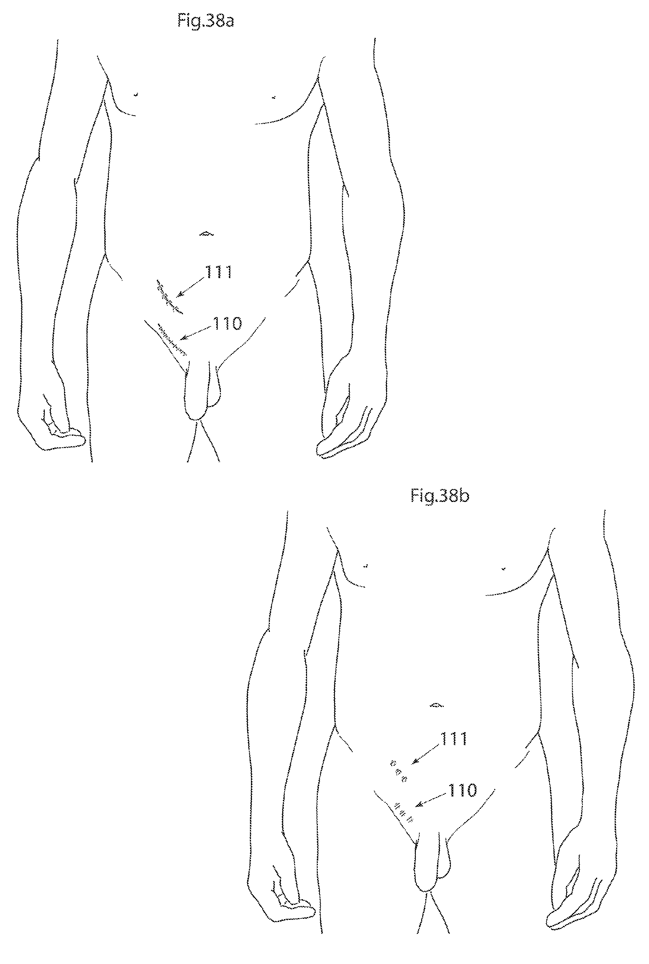

Fig. 38a shows the step of suturing 110 or stapling 111 the skin of the human patient in the surgical method, whereas fig. 38b shows the step of suturing 110 or stapling 111 the skin of the human patient in the laparoscopic method. The laparoscopic method may not need any suturing.

## Claims

1. A medical device for treating hip joint osteoarthritis in a human patient by providing at least one artificial hip joint surface, the hip joint comprising an acetabulum (11), being a part of the pelvic bone (9), and a caput femur (5) being a part of the femoral bone (7), and having a partly spherical form having a largest diameter, wherein said medical device comprises:
- a prosthetic part (98) or a bone plug (31) adapted to be placed in a hole (18) in the pelvic bone such that a concave interior surface of the prosthetic part or the bone plug is facing in a direction towards the caput femur, and
- a supporting member (95; 99) connected to said prosthetic part or bone plug, wherein
- said prosthetic part or the bone plug is adapted to be inserted into said hole from the abdominal side of the acetabulum and oriented, during the insertion, such that the concave interior surface is facing in the direction towards the caput femur, and
- said supporting member is adapted to connect to the pelvic bone and transfer a load from the prosthetic part or bone plug to the pelvic bone, when implanted, wherein at least one of:
o the medical device comprises an artificial caput femur surface (45), and wherein a largest cross-sectional distance of said artificial caput femur surface is smaller than said hole, such that said artificial caput femur surface can pass through said hole, and
∘ the medical device comprises an artificial acetabulum surface (65), and wherein a largest cross-sectional distance of said artificial acetabulum surface is smaller than said hole, such that said artificial acetabulum surface can pass through said hole.

2. The medical device according to claim 1, wherein said medical device has a largest diameter, and wherein said largest diameter is adapted to be changed during an operation for treating hip joint osteoarthritis in a human patient by providing at least one artificial hip joint surface.

3. The medical device according to any one of preceding claims, wherein said medical device further comprises at least one of:
- a slit (66), allowing said change in said largest diameter, and
- an elastic member or elastic part, allowing said change in said largest diameter.

4. The medical device according to any one of preceding claims, wherein said medical device comprises at least two parts (46; 68; 69), and wherein said at least two parts are adapted to be connected to each other after insertion in a hip joint of a human patient to form a functional artificial hip joint surface.

5. The medical device according to any one of preceding claims, wherein said medical device comprises at least one of:
- an artificial caput femur surface having a largest diameter,
- an artificial acetabulum surface having a largest diameter, and
- an artificial caput femur surface and an artificial acetabulum surface.

6. The medical device according to claim 5, wherein:
- said artificial acetabulum surface comprises at least one supporting member,
wherein:
- said at least one supporting member is adapted to be in connection with the pelvic bone after insertion through said hole in the pelvic bone, and
- said at least one supporting member is further adapted to carry the load placed on caput femur from the weight of said human patient by the connection with the pelvic bone, after insertion through said hole in the pelvic bone.

7. The medical device according to any one of the preceding claims, wherein said supporting member comprises at least one element selected from a group consisting of:
- screws (101),
- adhesive,
- at least one plate (95),
- bone cement, and
- a section of said artificial acetabulum.

8. The medical device according to any one of preceding claims, wherein said at least one supporting member is positioned on at least one of:
a, the abdominal side or the pelvic bone for directly or indirectly carrying said load, and
b. the acetabulum side of the pelvic bone for directly or indirectly carrying said load.

9. The medical device according to any one or preceding claims, wherein said at least one supporting member is in connection with the bone surrounding said hole, and wherein said bone directly or indirectly carries said load.

10. The medical device according to any one of preceding claims, wherein said at least one supporting member is adapted to be at least one of:
- fixated to the cortex of said pelvic bone for carrying said load, and
- fixated to the surface of said pelvic bone for carrying said load.

11. The medical device according to any one of preceding claims, wherein said medical device comprises a first and second part, and wherein said second part (95; 97) comprises said supporting member,

12. The medical device According to claim 11, wherein said second part is:
displaceable in relation to said first part,
- adapted to carry said load by the connection with the pelvic bone, and
- carries said load when displaced.

13. The medical device according to any one of preceding claims, wherein said at least one supporting member is at least one of:
- in connection with the bone surrounding said hole, and wherein the bone directly or indirectly carries said load,
- adapted to be fixated to the cortex of the pelvic bone for carrying said load, and
- adapted to be fixated to the surface of the pelvic bone for carrying said load.

14. The medical device according to any one of preceding claims, wherein the supporting member can be arranged in a first position allowing the medical device to pass through the hole in the pelvic bone, and in a second position in which the supporting member connects the medical device to the pelvic bone such that the medical device is allowed to transfer load from the prosthetic part or bone plug to the pelvic bone.

15. The medical device according to claim 14, wherein the supporting member is configured to be rotated so as to change from said first to said second position.

## Patentansprüche

1. Medizinische Vorrichtung zur Behandlung von Hüftgelenk-Osteoarthritis eines menschlichen Patienten durch Bereitstellung mindestens einer künstlichen Hüftgelenkfläche, wobei das Hüftgelenk ein Acetabulum (11), das ein Teil des Hüftknochens (9) ist, und einen Oberschenkelknochenkopf (5), der ein Teil eines Oberschenkelknochens (7) ist, umfasst und eine teilweise kugelförmige Form mit einem größten Durchmesser hat, wobei die medizinische Vorrichtung Folgendes umfasst:
- einen Protheseteil (98) oder einen Knochenzapfen (31), der geeignet ist, in ein Loch (18) im Hüftknochen platziert zu werden, so dass eine konkave innere Fläche des Protheseteils oder des Knochenzapfens in eine Richtung zum Oberschenkelknochenkopf hin gerichtet ist, und
- ein Stützglied (95; 99), das mit dem Protheseteil oder dem Knochenzapfen verbunden ist, wobei
- der Protheseteil oder der Knochenzapfen geeignet ist, von der Unterleibseite des Acetabulums in das Loch eingeführt und während des Einführens so ausgerichtet zu werden, dass die konkave innere Fläche in die Richtung zum Oberschenkelknochenkopf hin gerichtet ist, und
- das Stützglied geeignet ist, sich mit dem Hüftknochen zu verbinden und eine Last vom Protheseteil oder vom Knochenzapfen zum Hüftknochen zu übertragen, wenn es implantiert ist, wobei
• die medizinische Vorrichtung eine künstliche Oberschenkelknochenkopffläche (45) umfasst und wobei ein größter Querschnittsabstand der künstlichen Oberschenkelknochenkopffläche kleiner als das Loch ist, so dass die künstliche Oberschenkelknochenkopffläche durch das Loch gehen kann, und/oder
• die medizinische Vorrichtung eine künstliche Acetabulumfläche (65) umfasst und wobei ein größter Querschnittsabstand der künstlichen Acetabulumfläche kleiner als das Loch ist, so dass die künstliche Acetabulumfläche durch das Loch gehen kann.

2. Medizinische Vorrichtung nach Anspruch 1, wobei die medizinische Vorrichtung einen größten Durchmesser hat und wobei der größte Durchmesser geeignet ist, während einer Operation zur Behandlung von Hüftgelenk-Osteoarthritis eines menschlichen Patienten durch Bereitstellung mindestens einer künstlichen Hüftgelenkfläche geändert zu werden.

3. Medizinische Vorrichtung nach einem der vorhergehenden Ansprüche, wobei die medizinische Vorrichtung ferner
- einen Schlitz (66), der die Änderung bei dem größten Durchmesser gestattet, und/oder
- ein elastisches Glied oder einen elastischen Teil, das bzw. der die Änderung bei dem größten Durchmesser gestattet,
umfasst.

4. Medizinische Vorrichtung nach einem der vorhergehenden Ansprüche, wobei die medizinische Vorrichtung mindestens zwei Teile (46; 68; 69) umfasst und wobei die mindestens zwei Teile geeignet sind, nach Einführung in ein Hüftgelenk eines menschlichen Patienten miteinander verbunden zu werden, um eine funktionale künstliche Hüftgelenkfläche zu bilden.

5. Medizinische Vorrichtung nach einem der vorhergehenden Ansprüche, wobei die medizinische Vorrichtung
- eine künstliche Oberschenkelknochenkopffläche mit einem größten Durchmesser und/oder
- eine künstliche Acetabulumfläche mit einem größten Durchmesser und/oder
- eine künstliche Oberschenkelknochenkopffläche und eine künstliche Acetabulumfläche
umfasst.

6. Medizinische Vorrichtung nach Anspruch 5, wobei
- die künstliche Acetabulumfläche mindestens ein Stützglied umfasst, wobei
- das mindestens eine Stützglied geeignet ist, nach dem Einführen durch das Loch im Hüftknochen in Verbindung mit dem Hüftknochen zu sein, und
- das mindestens eine Stützglied nach dem Einführen durch das Loch im Hüftknochen ferner geeignet ist, durch die Verbindung mit dem Hüftknochen die durch das Gewicht des menschlichen Patienten auf den Oberschenkelknochenkopf platzierte Last zu tragen.

7. Medizinische Vorrichtung nach einem der vorhergehenden Ansprüche, wobei das Stützglied mindestens ein aus einer Gruppe ausgewähltes Element umfasst, wobei die Gruppe aus Folgendem besteht:
- Schrauben (101),
- Klebstoff,
- mindestens eine Platte (95),
- Knochenzement und
- eine Sektion des künstlichen Acetabulums.

8. Medizinische Vorrichtung nach einem der vorhergehenden Absprüche, wobei das mindestens eine Stützglied an
a. der Unterleibseite des Hüftknochens positioniert ist, um die Last direkt oder indirekt zu tragen, und/oder
b. der Acetabulumseite des Hüftknochens positioniert ist, um die Last direkt oder indirekt zu tragen.

9. Medizinische Vorrichtung nach einem der vorhergehenden Ansprüche, wobei das mindestens eine Stützglied mit dem das Loch umgebenden Knochen in Verbindung steht und wobei der Knochen die Last direkt oder indirekt trägt.

10. Medizinische Vorrichtung nach einem der vorhergehenden Ansprüche, wobei das mindestens eine Stützglied geeignet ist,
- an der Kortikalis des Hüftknochens fixiert zu sein, um die Last zu tragen, und/oder
- an der Oberfläche des Hüftknochens fixiert zu sein, um die Last zu tragen.

11. Medizinische Vorrichtung nach einem der vorhergehenden Ansprüche, wobei die medizinische Vorrichtung einen ersten und einen zweiten Teil umfasst und wobei der zweite Teil (95; 97) das Stützglied umfasst.

12. Medizinische Vorrichtung nach Anspruch 11, wobei der zweite Teil
- bezüglich des ersten Teils verschiebbar ist,
- geeignet ist, durch die Verbindung mit dem Hüftknochen die Last zu tragen, und
- bei Verschiebung die Last trägt.

13. Medizinische Vorrichtung nach einem der vorhergehenden Ansprüche, wobei das mindestens eine Stützglied
- mit dem das Loch umgebenden Knochen in Verbindung steht, und wobei der Knochen die Last direkt oder indirekt trägt, und/oder
- geeignet ist, an der Kortikalis des Hüftknochens fixiert zu sein, um die Last zu tragen, und/oder
- geeignet ist, an der Oberfläche des Hüftknochens fixiert zu sein, um die Last zu tragen.

14. Medizinische Vorrichtung nach einem der vorhergehenden Ansprüche, wobei das Stützglied in einer ersten Position, in der die medizinische Vorrichtung durch das Loch im Hüftknochen gehen kann, und in einer zweiten Position angeordnet sein kann, in der das Stützglied die medizinische Vorrichtung mit dem Hüftknochen verbindet, so dass die medizinische Vorrichtung Last vom Protheseteil oder vom Knochenzapfen auf den Hüftknochen übertragen kann.

15. Medizinische Vorrichtung nach Anspruch 14, wobei das Stützglied dazu konfiguriert ist, gedreht zu werden, um aus der ersten in die zweite Position zu wechseln.

## Revendications

1. Dispositif médical pour le traitement de l'ostéo-arthrite de l'articulation de la hanche chez un patient humain, en procurant au moins une surface artificielle de l'articulation de la hanche, l'articulation de la hanche comprenant un acétabulum (11), qui est une partie de l'os pelvien (9), et une tête de fémur (5) faisant partie de l'os fémoral (7), et ayant une forme partiellement sphérique présentant le plus grand diamètre, dans lequel ledit dispositif médical comprend:
- une partie prothétique (98) ou un bouchon osseux (31) apte à être placé (e) dans un trou (18) dans l'os pelvien de telle manière qu'une surface intérieure concave de la partie prothétique ou du bouchon osseux soit orientée en direction de la tête de fémur, et
- un élément de support (95; 99) connecté à ladite partie prothétique ou audit bouchon osseux, dans lequel
- ladite partie prothétique ou ledit bouchon osseux est apte à être inséré (e) dans ledit trou à partir du côté abdominal de l'acétabulum et orienté(e), pendant l'insertion, de telle manière que la surface intérieure concave soit orientée en direction de la tête de fémur, et
- ledit élément de support est apte à se connecter à l'os pelvien et à transférer une charge de la partie prothétique ou du bouchon osseux à l'os pelvien, lorsqu'il est implanté, dans lequel on a au moins un parmi:
o le dispositif médical comprend une surface de tête de fémur artificielle (45), et dans lequel la plus grande distance transversale de ladite surface de tête de fémur artificielle est plus petite que ledit trou, de telle manière que ladite surface de tête de fémur artificielle puisse passer à travers ledit trou, et
o le dispositif médical comprend une surface d'acétabulum artificiel (65), et dans lequel la plus grande distance transversale de ladite surface d'acétabulum artificiel est plus petite que ledit trou, de telle manière que ladite surface d'acétabulum artificiel puisse passer à travers ledit trou.

2. Dispositif médical selon la revendication 1, dans lequel ledit dispositif médical a un plus grand diamètre, et dans lequel ledit plus grand diamètre est apte à être modifié pendant une opération pour traiter l'ostéo-arthrite de l'articulation de la hanche chez un patient humain en procurant au moins une surface artificielle de l'articulation de la hanche.

3. Dispositif médical selon l'une quelconque des revendications précédentes, dans lequel ledit dispositif médical comprend en outre au moins un parmi :
- une fente (66), permettant ladite modification dudit plus grand diamètre, et
- un élément élastique ou une partie élastique, permettant ladite modification dudit plus grand diamètre.

4. Dispositif médical selon l'une quelconque des revendications précédentes, dans lequel ledit dispositif médical comprend au moins deux parties (46; 68; 69), et dans lequel lesdites au moins deux parties sont aptes à être connectées l'une à l'autre après insertion dans une articulation de la hanche chez un patient humain afin de former une surface fonctionnelle artificielle de l'articulation de la hanche.

5. Dispositif médical selon l'une quelconque des revendications précédentes, dans lequel ledit dispositif médical comprend au moins une de:
- une surface de tête de fémur artificielle ayant un plus grand diamètre,
- une surface d'acétabulum artificiel ayant un plus grand diamètre, et
- une surface de tête de fémur artificielle et une surface d'acétabulum artificiel.

6. Dispositif médical selon la revendication 5, dans lequel:
- ladite surface d'acétabulum artificiel comprend au moins un élément de support, dans lequel:
- ledit au moins un élément de support est apte à être en connexion avec l'os pelvien après insertion à travers ledit trou dans l'os pelvien, et
- ledit au moins un élément de support est en outre apte à porter la charge appliquée à la tête de fémur en raison du poids dudit patient humain par la connexion avec l'os pelvien, après insertion à travers ledit trou dans l'os pelvien.

7. Dispositif médical selon l'une quelconque des revendications précédentes, dans lequel ledit élément de support comprend au moins un élément sélectionné dans un groupe composé de:
- vis (101),
- adhésif,
- au moins une plaque (95),
- du ciment osseux, et
- une section dudit acétabulum artificiel.

8. Dispositif médical selon l'une quelconque des revendications précédentes, dans lequel ledit au moins un élément de support est positionné sur au moins un parmi:
a. le côté abdominal de l'os pelvien afin de porter directement ou indirectement ladite charge, et
b. le côté acétabulaire de l'os pelvien afin de porter directement ou indirectement ladite charge.

9. Dispositif médical selon l'une quelconque des revendications précédentes, dans lequel ledit au moins un élément de support est en connexion avec l'os entourant ledit trou, et dans lequel ledit os porte directement ou indirectement ladite charge.

10. Dispositif médical selon l'une quelconque des revendications précédentes, dans lequel ledit au moins un élément de support est apte à être au moins un parmi:
- fixé au cortex dudit os pelvien pour porter ladite charge, et
- fixé à la surface dudit os pelvien pour porter ladite charge.

11. Dispositif médical selon l'une quelconque des revendications précédentes, dans lequel ledit dispositif médical comprend une première et une deuxième parties, et dans lequel ladite deuxième partie (95; 97) comprend ledit élément de support.

12. Dispositif médical selon la revendication 11, dans lequel ladite deuxième partie est:
- déplaçable par rapport à ladite première partie,
- apte à porter ladite charge par la connexion avec l'os pelvien, et
- porte ladite charge lorsqu'elle est déplacée.

13. Dispositif médical selon l'une quelconque des revendications précédentes, dans lequel ledit au moins un élément de support se trouve dans au moins un des cas suivants:
- en connexion avec l'os entourant ledit trou, et dans lequel l'os porte directement ou indirectement ladite charge,
- apte à être fixé au cortex de l'os pelvien pour porter ladite charge, et
- apte à être fixé à la surface de l'os pelvien pour porter ladite charge.

14. Dispositif médical selon l'une quelconque des revendications précédentes, dans lequel l'élément de support peut être agencé dans une première position permettant au dispositif médical de passer à travers le trou dans l'os pelvien, et dans une deuxième position dans laquelle l'élément de support connecte le dispositif médical à l'os pelvien de telle manière que le dispositif médical puisse transférer une charge de la partie prothétique ou du bouchon osseux à l'os pelvien.

15. Dispositif médical selon la revendication 14, dans lequel l'élément de support est configuré de façon à tourner pour changer de ladite première à ladite deuxième position.
